# EUROPÄISCHE PATENTANMELDUNG

(11) **EP 2 869 066 A1**
(43) Veröffentlichungstag der Anmeldung: **06.05.2015**
(21) Anmeldenummer: 14188758.8
(22) Anmeldetag: 14.10.2014
(51) Int. Cl.: G01N 27/06, A47J 31/60, G01N 33/14

(54) **Verfahren zur Erkennung von Reinigungsmitteln in einer Getränkemaschine, insbesondere einer Kaffeemaschine**

(30) Priorität: 05.11.2013 DE 102013112130
(71) Anmelder: Melitta Professional Coffee Solutions GmbH & Co., KG, 32429 Minden (DE)
(72) Erfinder: Buchholz, Bernd, 32369 Rahden (DE); Hallmann, Kai, 32423 Minden (DE)
(74) Vertreter: Specht, Peter

(57) **Zusammenfassung**

Die vorliegende Erfindung betrifft ein Verfahren zum Reinigen eines HeißGetränkemaschine, insbesondere einer Kaffeemaschine, insbesondere eines Kaffeeautomaten, bei dem ein, insbesondere für die Messung des Füllstandes eines Getränkezusatzbehälters genutzter, konduktiver Leitwert- Durchflusssensor vorzugsweise zudem genutzt wird, um die Konzentration, und gegebenenfalls zudem den pH-Wert, eines in einer Reinigungsflüssigkeit gelösten Reinigungsmittels zu messen.

## Beschreibung

Die Erfindung betrifft ein Verfahren zum Erkennen von Reinigungsmittel in Abschnitten, insbesondere in von Heiß- oder Kaltgetränk, insbesondere von Milch, durchflossenen Abschnitten, insbesondere Leitungen, einer Getränkemaschine, vorzugsweise einer Kaffeemaschine.

Bei der Getränkeherstellung, insbesondere bei der Kaffeeherstellung unter Zusatz von Milch oder Milchschaum, müssen der oder die von Heiß- oder Kaltgetränk durchflossenen Bereiche der Getränkemaschine, insbesondere die von Milch durchflossenen Bereich, vorzugsweise Leitung(en), regelmäßig sorgfältig mit einer Reinigungsflüssigkeit gereinigt werden. Als Reinigungsflüssigkeit wird dafür zumeist Wasser verwendet, in dem ein Reinigungsmittel aufgelöst wurde. Um eine ausreichende Reinigung in insbesondere von Heiß- und/oder Kaltgetränk, insbesondere von oder mit Milch durchflossenen Bereichen zu gewährleisten, muss dem Wasser eine ausreichende Menge Reinigungsmittel zugesetzt werden. "Von Heiß- und/oder Kaltgetränk", insbesondere "von Milch durchflossene" Bereiche bedeutet im Zusammenhang der Beschreibung, der Ansprüche und der Figuren dieses Schutzrechtes, dass allein Kaffee, Tee, Wasser, Schokolade/Kakao oder Milch oder Milch als Teil eines Heiß- oder Kaltgetränkes durch den oder die Bereiche fließt.

Aufgabe der Erfindung ist es, ein Verfahren zu schaffen, mit dem auf einfache Weise überprüft werden kann, ob in dem oder den von Getränk durchflossenen Bereich(en) Reinigungsmittel, so beispielsweise Reinigungsmittelrückstände, vorhanden sind.

Diese Aufgabe löst die Erfindung durch den Gegenstand des Anspruchs 1. Vorteilhafte Ausgestaltungen sind den Unteransprüchen zu entnehmen.

Vorzugsweise wird mit einem konduktiven Leitwert-Durchflusssensor die auf der Beweglichkeit von elektrischen Ladungsträgern wie Elektronen und Ionen basierende Leitfähigkeit des Reinigungsmittels ermittelt, um derart die Menge des im Wasser gelösten Reinigungsmittels zu bestimmen.

Es ist zwar bekannt, in Kaffeemaschinen, beispielweise in Kaffeevollautomaten den Füllstand eines Milchbehälters mit Hilfe eines konduktiven Leitwert- Durchflusssensors zu messen. Dabei wurde aber nicht erkannt, dass sich dieser Sensor auch dazu eignet, die wichtige weitere Funktion einer Reinigungsmittelerkennung zu übernehmen.

In einer weiteren bevorzugten Ausführungsform umfasst die Auswertung der Messung die Bestimmung von Eigenschaften des Reinigungsmittels. So kann über die Messung bestimmt werden, ob überhaupt Reinigungsmittel im System vorhanden ist. Es kann aber auch die Reinigungsmittelkonzentration ermittelt werden, um festzustellen, ob das Reinigungsmittel in der richtigen Dosierung vorhanden ist. Zudem ist auch eine Bestimmung des pH- Wertes des Reinigungsmittels zweckmäßig, durch welche festgestellt werden kann, ob basisches oder saures Reinigungsmittel verwendet wird.

Zudem ist es bevorzugt, dass für die Messung der ohnehin für die Füllstands- Messung, insbesondere für die Messung des geleert Seins, des Getränkezusatzbehälters vorgesehene konduktive Leitwert- Durchflusssensor genutzt wird. Der Heißgetränkeautomat benötigt daher keinen zusätzlichen konduktiven Leitwert- Durchflusssensor.

Um die Genauigkeit des Verfahrens zu verbessern, ist es außerdem bevorzugt, dass die Temperatur der Reinigungsflüssigkeit gemessen und bei der Berechnung der Konzentration des Reinigungsmittels und/oder des pH- Wertes berücksichtigt wird, um die Temperaturabhängigkeit des Leitwertes zu kompensieren.

Im Folgenden ist die Erfindung anhand von Figuren beschrieben. Die Figuren sind lediglich beispielhaft und schränken den allgemeinen Erfindungsgedanken nicht ein. Es zeigt
- Fig. 1: schematisch den Aufbau einer Getränkemaschine, insbesondere einer Kaffeemaschine, und
- Fig. 2: das Ergebnis einer Leitfähigkeitsmessung in einer Zuleitung der Getränkemaschine, insbesondere der Kaffeemaschine.

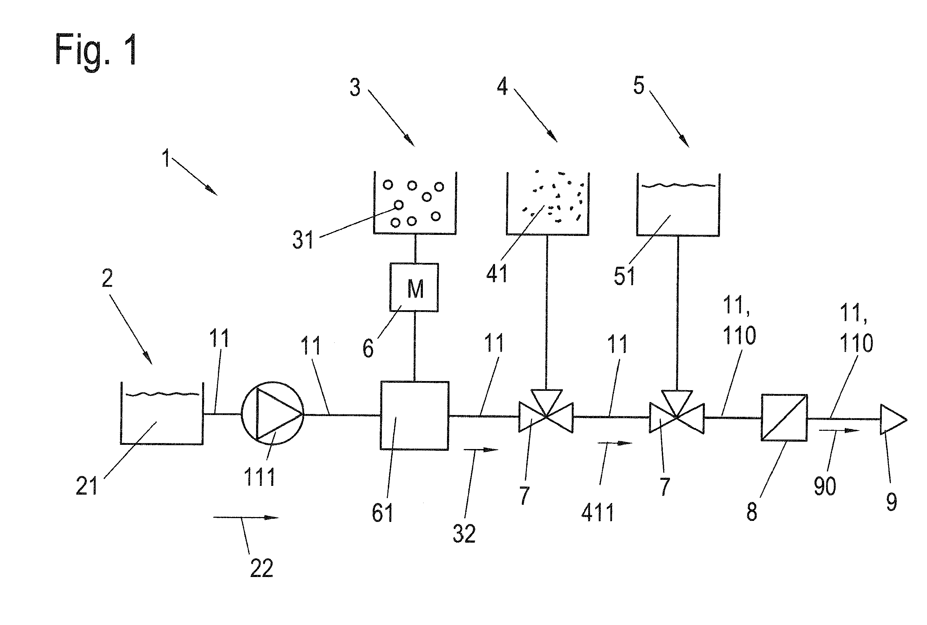

In der Fig. 1 ist schematisch eine vereinfacht dargestellte Getränkemaschine 1 dargestellt. Die dargestellte Getränkemaschine 1 ist eine Kaffeemaschine. Im Folgenden wird für die Getränkemaschine 1 daher der Begriff Kaffeemaschine verwendet. Die Erfindung ist aber auch bei anderen Getränkemaschinen wie Teemaschinen, Schokoladenmaschinen usw. anwendbar.

Um Kaffee 32 herzustellen, weist die Kaffeemaschine 1 einen Wasserbehälter 2 mit Wasser 21 oder eine Wasserzuleitung für Wasser 21 auf. Vorgesehen ist ferner ein Rohstoffbehälter 3 mit einem Rohstoff 31. Als Rohstoff sind hier beispielhaft Kaffeebohnen dargestellt. Es ist aber auch eine sonstige Rohstoffversorgung denkbar, beispielsweise über Kapseln. Vorgesehen sind zudem ein Reinigungsbehälter 4 mit einem Reinigungsmittel 41 und optional ein Getränkezusatzbehälter 5 mit einem Getränkezusatz 51.

Die Herstellung des Kaffees 32 erfolgt während eines Brühvorganges. Das fertige Kaffeegetränk 32 ist hier durch einen Pfeil dargestellt. Es durchfließt das Rohrsystem 11 nach dem Brühvorgang, nicht aber während eines Reinigungsvorgangs, und gegebenenfalls auch nicht während eines Ablassvorgangs für den Getränkezusatz.

Bei dem Kaffeezubereitungsvorgang werden die Kaffeebohnen 31 in einem Mahlwerk 6 gemahlen. Wasser 21 wird sodann in einer Brühgruppe 61 unter Druck durch das gepresste Kaffeemehl geleitet. Anschließend wird der Kaffee über ein Ablaufventil 9 aus dem Kaffeeautomaten 1 in einen geeigneten Behälter (nicht gezeigt), beispielsweise eine Tasse, abgelassen.

Um Getränkevarianten herstellen zu können, weist der Kaffeeautomat 1 einen Getränkezusatzbehälter 5 auf, die hier für Milch 51 vorgesehen ist. Die Milch 51 kann-ggf. geeignet aufgeschäumt - vor, während oder nach dem Durchlauf des Kaffees 32 durch das Ablaufventil 9 zulaufen.

Rein schematisch ist der Reinigungsbehälter 4 mit dem Reinigungsmittel 41 dargestellt. Das Reinigungsmittel 41 wird dem Wasser 21 im Rahmen eines Reinigungsvorgangs zugesetzt. Das Reinigungsmittel 41 kann beispielsweise dem durch die Brühgruppe 61 geleiteten Wasser 21 hinter oder vor der Brühgruppe 61 zugesetzt werden oder aber an anderer Stelle der Kaffeemaschine, beispielsweise am Behälter 5 (nicht dargestellt). Es ist auch denkbar, eine vorgefertigte Reinigungsflüssigkeit zuzusetzen.

Die aus dem Wasser und einem Reinigungsmittel bestehende Reinigungsflüssigkeit 411 ist schematisch durch einen Pfeil dargestellt. Sie durchfließt während des Reinigungsvorgangs einen Abschnitt der Kaffeemaschine. Vorzugsweise weist die Kaffeemaschine 1 einen Reinigungskreislauf (nicht gezeigt) auf, in dem die Reinigungsflüssigkeit 411 mehrfach wieder durch den zu reinigenden Bereich geleitet wird, wozu vorzugsweise jedenfalls sämtliche von Milch durchströmten Abschnitte gehören (hier vereinfacht anders dargestellt).

Sodann wird die Reinigungsflüssigkeit 411 nach dem Durchfließen der von Milch 51 durchflossenen Abschnitte 110 durch das Ablaufventil 9 abgelassen.

Der dargestellte Kaffeeautomat 1 weist einen konduktiven Leitwert- Durchflusssensor 8 auf, der in einer mit der Milch 51 durchflossenen Zuleitung 110 angeordnet ist, und zwar hier in der zum Ablaufventil 9 führenden Zuleitung 110. Diese Zuleitung 110 ist die letzte Zuleitung 110 vor dem Ablaufventil 9, so dass neben dem Ablaufventil 9 alle zwischen dem Reinigungsbehälter 4 und dem Ablaufventil 9 angeordneten Zuleitungen 11, 110 und Ventile 7 im Rahmen des Reinigungsvorgangs gereinigt werden. Andere Ausgestaltungen sind denkbar. Die Darstellung ist insofern nur rein beispielhaft zu verstehen.

Gemäß dem Stand der Technik wird mit diesem konduktiven Leitwert- Durchflusssensor 8 die Leitfähigkeit der ihn durchfließenden Flüssigkeit 90 gemessen, um den Füllstand des Milchbehälters 5 zu bestimmen. Dabei wird konkret gemessen, ob noch Milch 5 im Milchbehälter 51 vorhanden ist, oder nicht. Gemäß dem Stand der Technik erfolgt diese Messung während eines Ablassvorgangs , bei dem die Milch 5 oder milchhaltige Flüssigkeit 90 über das Ablaufventil 9 abgelassen wird, und/oder während eines Kaffeezubereitungsvorganges, bei dem milchhaltiger Kaffee 32 über das Ablaufventil 9 abgelassen wird.

In der gezeigten Ausführungsform wird dieser konduktive Leitwert- Durchflusssensor 8 zudem genutzt, um die Konzentration des in der abgelassenen Flüssigkeit 90 gelösten Reinigungsmittels 41 festzustellen. Die Messung erfolgt daher nicht nur während des Ablassvorgangs der Milch 5 oder milchhaltigen Flüssigkeit, sondern zudem und vor allem während des Reinigungsvorgangs.

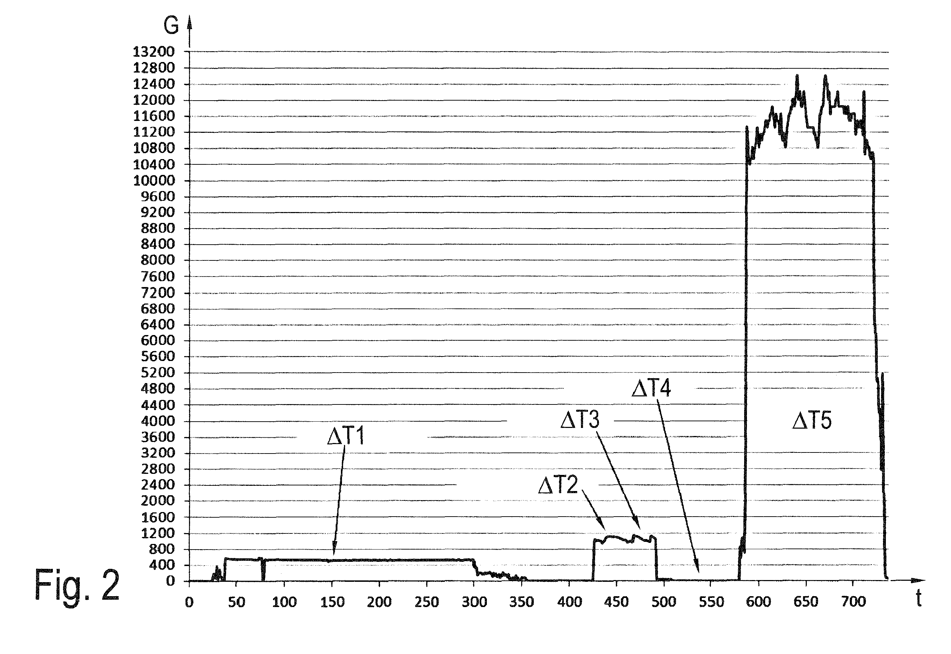

Eine solche Messung eines Leitwertes G der abgelassenen Flüssigkeit 90 während eines Reinigungsvorgangs zeigt die Fig. 2.

Das Diagramm zeigt den gemessenen Leitwert G in µS (Siemens) auf der Ordinate gegenüber der Zeit in Millisekunden auf der Abszisse.

In einem ersten Zeitraum ΔT1 wird Kaltwasser durch das Rohrsystem 11 in die Zuleitung 110 und durch den konduktiven Leitwert- Durchflusssensor 8 geführt, und durch das Ablaufventil 9 abgelassen.

In einem zweiten Zeitraum ΔT2 wird Warmwasser durch das Rohrsystem 11 in die Zuleitung 110 und durch den konduktiven Leitwert- Durchflusssensor 8 geführt, und durch das Ablaufventil 9 abgelassen.

In einem dritten Zeitraum ΔT3 wird der Mischbehälter mit Wasser 21 gefüllt.

In einem vierten Zeitraum ΔT4 wird das Reinigungsmittel 41 in den Mischbehälter gefüllt und zur Reinigungsflüssigkeit 411 aufgelöst.

Und in einem fünften Zeitraum ΔT5 wird die Reinigungsflüssigkeit 41 durch das Rohrsystem 11 in die Zuleitung 110 und durch den konduktiven Leitwert- Durchflusssensor 8 geführt, und durch das Ablaufventil 9 abgelassen.

In einem sechsten, hier nicht dargestellten Zeitraum wird nochmals Kaltwasser durch das Rohrsystem 11 in die Zuleitung 110 und durch den konduktiven Leitwert- Durchflusssensor 8 geführt, und durch das Ablaufventil 9 abgelassen, um die von der Reinigungsflüssigkeit 411 durchflossenen Zuleitungen 11, 110 und Ventile 7, 9 von dieser zu reinigen.

Sichtbar ist, dass das im ersten Zeitraum ΔT1 fließende Kaltwasser 21 den kleinsten Leitwert G aufweist. Dieser liegt im Bereich 400 - 800 µS. Das erwärmte, im zweiten Zeitraum ΔT2 fließende Wasser 21 weist aufgrund der Temperaturabhängigkeit des Leitwertes G von Wasser 21 einen geringfügig höheren Leitwert G von 800 - 1200 µS auf.

Der im fünften Zeitraum ΔT5 gemessene Leitwert G der Reinigungsflüssigkeit 411 ist erheblich höher, als der des kalten oder warmen Wassers 21. Er liegt im Bereich zwischen 10400 und 12800 µS.

Der Leitwert G von geeigneten Reinigungsflüssigkeiten 411 kann variieren. Je nach Getränk 21 oder Getränkezusatz 51 werden Reinigungsmittel 41 mit verschiedenen, eher sauren oder eher basischen pH- Werten verwendet. Zudem variiert der gemessene Leitwert mit der Konzentration des Reinigungsmittels 41 in der Reinigungsflüssigkeit 411, d. h. mit der im Wasser 21 gelösten Menge Reinigungsmittel 41.

Der Kaffeeautomat 1 weist daher eine Steuerung (nicht gezeigt) auf, die eine Auswerteeinheit (nicht gezeigt) umfasst, welche dazu ausgelegt ist, den gemessenen Leitwert G mit Referenzwerten zu vergleichen. Für den Vergleich werden herkömmliche Verfahren eingesetzt. Die Referenzwerte sind bevorzugt in einem Datenspeicher (nicht gezeigt) des Kaffeeautomaten 1 gespeichert.

Ist nicht mehr genügend Reinigungsmittel 41 im Reinigungsbehälter 4 vorhanden, so dass die Reinigung unzureichend ist, und der gemessene Leitwert G zu niedrig ist, wird dies bevorzugt an einem Anzeigemittel (nicht gezeigt) wie beispielsweise einem Display oder einer LED (Leuchtdiode) angezeigt. Ergänzend ist es beispielsweise zur Fehlerauswertung vorteilhaft, wenn einer oder mehrere Reinigungsmittel-Konzentrationswerte protokolliert werden, d.h., vorzugsweise in einer Speichereinheit der Steuerung abgespeichert werden. Der Reinigungsvorgang wird dann nach dem Auffüllen des Reinigungsbehälters 4 wiederholt.

**Bezugszeichenliste**

| | |
|---|---|
| 1 | Heißgetränkeautomat, Kaffeeautomat |
| 2 | Wasserbehälter |
| 21 | Wasser |
| 22 | Durchflussrichtung |
| 3 | Rohstoffbehälter, Kaffeebehälter |
| 31 | Rohstoff, Kaffeebohnen |
| 32 | Getränk, Kaffee |
| 4 | Reinigungsbehälter |
| 41 | Reinigungsmittel |
| 411 | Reinigungsflüssigkeit |
| 5 | Getränkezusatzbehälter, Milchbehälter |
| 51 | Getränkezusatz, Milch |
| 6 | Mahlwerk |
| 61 | Brühgruppe |
| 7 | Ventile |
| 8 | Konduktiver Leitwert- Duchflusssensor |
| 9 | Ablaufventil |
| 90 | die Zuleitung 110 / das Ablaufventil durchfließende Flüssigkeit |
| 11 | Rohrsystem |
| 110 | Zuleitung |
| 111 | Pumpe |
| G | Leitwert |
| t | Zeit |
| ΔT1 - ΔT5 | Erster - fünfter Zeitraum |

## Patentansprüche

1. Verfahren zum Erkennen von Reinigungsmittel in Abschnitten (110), insbesondere in von Heiß- oder Kaltgetränk, insbesondere von Milch, durchflossenen Abschnitten (110), insbesondere Leitungen, einer Getränkemaschine, vorzugsweise einer Kaffeemaschine (1), **dadurch gekennzeichnet, dass** mit einem Leitwert-Durchflusssensor (8) der Kaffeemaschine (1) und einer daran angeschlossenen Auswertungseinrichtung ermittelt wird, ob die am Leitwert- Durchflusssensor (8) vorbeiströmende Flüssigkeit ein Reinigungsmittel enthält.

2. Verfahren nach Anspruch 1, **dadurch gekennzeichnet, dass** mit dem Leitwert-Durchflusssensor (8) der Kaffeemaschine (1) und der daran angeschlossenen Auswertungseinrichtung ermittelt wird, wie hoch die Konzentration an Reinigungsmittel (41) in der Flüssigkeit ist.

3. Verfahren nach Anspruch 1 oder 2, **dadurch gekennzeichnet, dass** mit dem Leitwert-Durchflusssensor (8) der Kaffeemaschine (1) und der daran angeschlossenen Auswertungseinrichtung ermittelt wird, ob die Konzentration an Reinigungsmittel (41) von einem vorgegebenen Schwellwert abweicht.

4. Verfahren nach Anspruch 1, 2, 3 oder 4, **dadurch gekennzeichnet, dass** als Leitwert- Durchflusssensor (8) ein konduktiver Leitwert- Durchflusssensor (8) verwendet wird.

5. Verfahren nach einem der vorstehenden Ansprüche, **dadurch gekennzeichnet, dass** der konduktive Leitwert- Durchflusssensor (8) zudem für die Messung des pH-Wertes der Reinigungsflüssigkeit (411) verwendet wird.

6. Verfahren nach einem der vorherigen Ansprüche, **dadurch gekennzeichnet, dass** die Reinigungsflüssigkeit (411) zumindest Wasser (21) und das Reinigungsmittel (41) enthält.

7. Verfahren nach einem der vorherigen Ansprüche, **dadurch gekennzeichnet, dass** die Temperatur der Reinigungsflüssigkeit gemessen wird.

8. Verfahren nach einem der vorherigen Ansprüche, **dadurch gekennzeichnet, dass** der konduktive Leitwert- Durchflusssensor (8) ein für die Messung des Füllstandes eines Getränkezusatzbehälters (5), insbesondere für das geleert Sein des Getränkezusatzbehälters (5), genutzter konduktiver Leitwert- Durchflusssensor (8) ist.

9. Verfahren nach einem der vorherigen Ansprüche, **dadurch gekennzeichnet, dass** es in einem Reinigungsvorgang der Getränkemaschine, insbesondere einer Kaffeemaschine (1), durchgeführt wird.

10. Verfahren nach einem der vorherigen Ansprüche, **dadurch gekennzeichnet, dass** der konduktive Leitwert- Durchflusssensor (8) in einer letzten Zuleitung (110) vor einem Ablaufventil (9) der Getränkemaschine, insbesondere einer Kaffeemaschine (1), angeordnet ist.

11. Verfahren nach einem der vorherigen Ansprüche, **dadurch gekennzeichnet, dass** die gemessenen Leitwerte (G) durch Vergleich mit Referenzwerten ausgewertet werden.

12. Verfahren nach einem der vorherigen Ansprüche, **dadurch gekennzeichnet, dass** die Referenzwerte temperaturabhängig sind.

13. Verfahren nach einem der vorherigen Ansprüche, **dadurch gekennzeichnet, dass** eine zu geringe gemessene Konzentration des Reinigungsmittels (41) in der Reinigungsflüssigkeit (411) an einem Anzeigemittel, insbesondere an einem Display oder einem Leuchtmittel, angezeigt wird.

14. Verfahren nach einem der vorherigen Ansprüche, **dadurch gekennzeichnet, dass** einer oder mehrere Reinigungsmittel-Konzentrationswerte in einer Speichereinheit abgespeichert werden.

15. Verwendung eines für die Messung des Füllstandes eines Getränkezusatzbehälters (5), insbesondere für das geleert Sein des Getränkezusatzbehälters (5), genutzten konduktiven Leitwert- Durchflusssensors (8) für die Messung einer Reinigungsmittelkonzentration in von Milch durchflossenen Abschnitten (110), insbesondere Leitungen, einer Getränkemaschine, vorzugsweise einer Kaffeemaschine (1).

16. Heißgetränkeautomat zur Durchführung des Verfahrens nach einem der vorherigen Ansprüche.
